# EUROPEAN PATENT SPECIFICATION

(11) **EP 2 646 010 B1**
(45) Date of publication and mention of the grant of the patent: **13.11.2019**
(21) Application number: 11724029.1
(22) Date of filing: 06.05.2011
(51) Int. Cl.: A61K 31/00, A61K 31/198, A61K 31/20, A61K 31/375, A61K 31/403, A61K 31/505, A61K 31/519, A61K 31/70, A61K 31/714, A61K 33/06, A61K 33/14

(54) **FOLIC ACID - RAMIPRIL COMBINATION: CELLPROTECTIVE, NEUROPROTECTIVE AND RETINOPROTECTIVE OPHTALMOLOGIC COMPOSITIONS**
FOLSÄURE -RAMIPRIL-KOMBINATION: ZELLSCHÜTZENDE, NERVENSCHÜTZENDE UND NETZHAUTSCHÜTZENDE ZUSAMMENSETZUNGEN FÜR DAS AUGE
COMBINAISON ACIDE FOLIQUE - RAMIPRIL : COMPOSITIONS OPHTALMOLOGIQUES CELLULO-PROTECTRICES, NEUROPROTECTRICES ET RÉTINOPROTECTRICES

(30) Priority: 03.12.2010 TN 10566
(43) Date of publication of application: 09.10.2013
(73) Proprietor: Rekik, Raouf, 1073 Tunis (TN)
(72) Inventor: Rekik, Raouf, 1073 Tunis (TN)
(74) Representative: Desaix, Anne
(86) International application number: PCT/IB2011/000960
(87) International publication number: WO 2012/073077

(56) References cited:
- WO-A1-2006/015489
- WO-A1-2007/134870
- WO-A1-2008/132756
- CN-A- 101 322 743
- CN-A- 101 590 084
- US-A1- 2002 164 388
- US-A1- 2003 049 314
- US-A1- 2006 106 057
- US-B1- 6 207 190
- SAHEL JOSÉ-ALAIN ET AL: "Clinical characteristics and current therapies for inherited retinal degenerations.", COLD SPRING HARBOR PERSPECTIVES IN MEDICINE 16 OCT 2014, vol. 5, no. 2, 16 October 2014 (2014-10-16), page a017111, ISSN: 2157-1422

## Description

### Field and background

Is disclosed a composition and, in particular, a cellprotective, neuroprotective and retinoprotective composition which is especially appropriate for maintaining or improving visual function (visual acuity and/or vision field). According to a particular aspect, said composition comprises (i) Ramipril or Ramiprilate and (ii) folic acid.

Is also disclosed the use of said composition for the prevention or the treatment of different types of disease(s) or disorder(s) and especially of cancers or of ophthalmologic disease(s) or disorders involving chorio-retinal and/or optic nerve, resulting in a progressive loss of vision (visual acuity and/or field of vision).

Such effects on vision are observed, in particular, in glaucoma, macular degeneration, inherited retinal diseases, myopia, presbyopia, uveitis and diabetic retinopathy. The invention is more particularly directed a composition for use in the prevention and/or treatment of a disease selected amongst pigmentosa retinopathy and Stargardt's disease, as defined in claims 1 to 8.

According to a particular aspect of the disclosure, a combination of angiotensin converting enzyme inhibitor (for example, ramipril or ramiprilate) and folic acid (or folate) and optionally one or several compounds chosen among vitamin B12, vitamin B6, Vitamin C, H4B (tetrahydrobiopterin), L-arginine, w-3 fatty acids (omega 3 fatty acids), magnesium, potassium, glucose and/or amino-acids (for example, a leucine, and especially an acetyl-leucine), in appropriate amounts, form a novel approach in the prevention and management of various diseases or disorders and, in particular, of ophthalmologic conditions such as glaucoma, glaucoma neuropathy, age related macular degeneration, inherited retinal dystrophies, myopia, presbyopia, diabetic retinopathy, or other diseases described herein.

Is disclosed the provision of compositions or medicaments (or drugs) and a kit intended for the prevention and/or the treatment of ophthalmologic diseases or disorders, which can improve visual function, in particular, the field of vision and/or visual acuity in patients suffering from neuropathies such as glaucoma, but also other chorioretinal disorders or deterioration of the optic nerve that may involve a vascular factor. Examples of ophthalmologic diseases that can be mentioned are:
1- Glaucomatous neuropathy, including glaucoma itself;
2- Degenerative chorio retinopathy;
3- Age-related macular degeneration (ARMD);
4- Hereditary dystrophies of the retina (including pigmentosa retinopathy and stargardt's maculopathy);
5- Retinal vascular occlusion ;
6- Myopia ;
7- Presbyopia ;
8- Uveitis ;
9- Diabetic retinopathy ;
10- Corneal disorders, for example, keratoconjunctivitis, dry eye syndrome, or Fuchs' corneal dystrophy; and
11- Physiologic visual degradation.

CN 101 322 743 A, US 6 207 190 B1 and US 2002/164388 A1 discuss treatments for ophthalmological conditions, which are respectively asthenopia, eyesight worsening or glaucoma, but these document do not disclose angiotensin converting enzyme inhibitors, and do not discuss pigmentosa retinopathy or Stargardt's disease.

WO 2006/015489 A1 generally relates to the use of particular angiotensin converting enzyme inhibitor, which is lisonopril and a particular vitamin B6 related compound for treating vascular disease in a diabetic patient. Folic acid is not disclosed in this document.

The invention is based, in particular, on the fact that when used to treat patients, Ramipril associated with a second particular active principle, for example, folic acid, causes not only an interruption - which until now has never been observed in patients treated for ophthalmologic diseases and especially diseases of the types mentioned herein - but moreover at least a partial inversion of the process of degradation of visual function, with an improvement in visual acuity and/or field of vision (usually both in visual acuity and field of vision).

In addition, it was found that folic acid, as well as other particular compounds and especially vitamin c, w-3 fatty acids and H4B augment the activity of Ramipril. Hence, the combination of Ramipril (or ramiprilate) with one or several of these active principles as disclosed herein is more efficient to prevent or to treat some ocular pathologies and presents a more cellprotective, neuroprotective and retinoprotective effect than each of these drugs used alone.

The combinations of active principles disclosed herein can be employed successfully to prevent or slow down or even stop a "natural" decrease in visual acuity, field of vision or both at the same time. In particular, they can be used successfully to prevent and even reverse visual decline in an aging animal (especially an aging human or non human mammal) and especially physiologic visual decline.

Mean retinal sensitivity reduces linearly with age. This decline starts very early on, from 20 years of age, and accelerates after the age of 60.

Ramipril, which is marketed, in particular, as Tritace®, Triatec®, Delix® or Altace®, has the following formula:

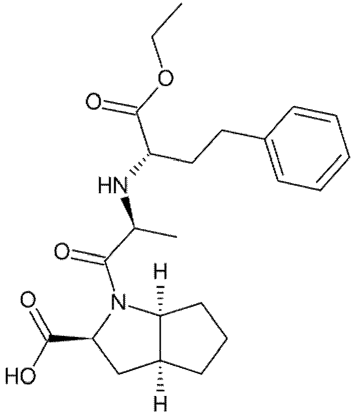

Ramiprilate, which results from de-esterification of ramipril, has the following formula:

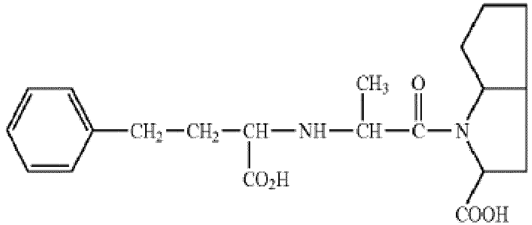

Folic Acid (also known as vitamin B9, vitamin M or folacin) has the following formula:

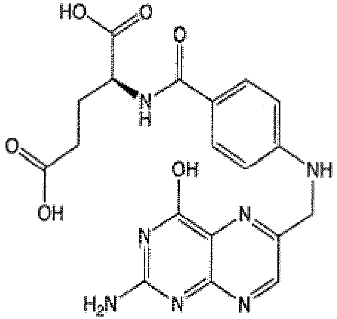

Folate (the naturally occurring form of folic acid) has the following formula:

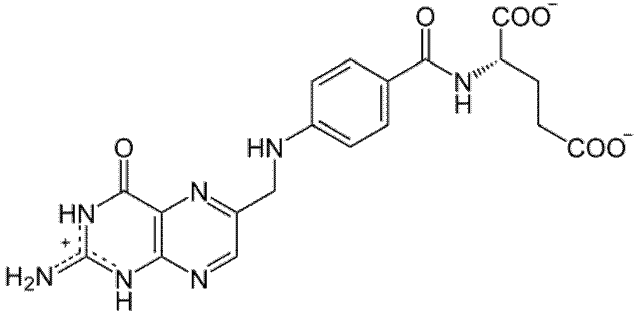

### Summary of the Invention

Is disclosed a composition comprising at least two active principles chosen among an angiotensin-converting enzyme inhibitor, folic acid, folate, magnesium, potassium, glucose, amino-acids (especially a leucine and, in particular, an acetyl-leucine), L-arginine, tetrahydrobiopterin (H4b), vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, their pharmaceutically acceptable salts, and mixtures thereof. In a particular aspect, the composition comprises (i) Ramipril or Ramiprilate and (ii) folic acid.

Such a composition can be used as medicament and, in particular, as a cellprotector, a neuroprotector and/or a retinoprotector medicament. More especially, is disclosed herein such a composition for use in the prevention and/or the treatment in an animal in need thereof of one or several disease(s) or disorder(s) chosen from: ophthalmologic conditions, cancers, arterial hypertension, hyperlipemia, coronary heart disease, atherosclerosis, diabete, neurodegenerative conditions (for example, multiple sclerosis, Alzheimer disease and/or Parkinson disease), rheumatism, general inflammatory and immune conditions and infections (for example, viral and especially HIV infection, bacterial infection and/or parasitic infections).

The invention more specifically relates to a composition for use in the prevention and/or the treatment of a disease selected amongst pigmentosa retinopathy and Stargardt's disease, characterized in that it comprises:
a. a first active principle, which consists of an angiotensin-converting enzyme inhibitor (ACEI) selected from the group consisting of: Ramipril, Ramiprilate, and one of their pharmaceutically acceptable salts, and
b. a second active principle that is selected from the group consisting of: folic acid, folate, and one of their pharmaceutically acceptable salts, and
c. optionally, at least a further active principle chosen among: magnesium, potassium, glucose, amino-acids, L-arginine, tetrahydrobiopterin (H4b), vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, their pharmaceutically acceptable salts, and mixtures thereof, as stated in claim 1.

Is also disclosed a composition for use for preventing and/or treating one or several disease(s) or disorder(s) in an animal in need thereof, and a composition for use for maintaining or improving vision and, in particular, the visual acuity and/or the field of vision in an animal in need thereof.

Is also disclosed a kit for use for preventing and/or treating, and especially to a kit comprising at least two active principles as disclosed herein, and, optionally, instructions for using said kit.

The invention more specifically relates to a kit for simultaneous or separate use in the prevention and/or the treatment of a disease selected amongst pigmentosa retinopathy and Stargardt's disease, as defined in claims 9 to 13.

According to a particular embodiment, the invention relates to a kit comprising:
a. a first active principle, which consists of an angiotensin-converting enzyme inhibitor (ACEI) selected from the group consisting of: Ramipril, Ramiprilate, and one of their pharmaceutically acceptable salts, and
b. a second active principle that is selected from the group consisting of: folic acid, folate, and
c. optionally, at least a further active principle chosen among: magnesium, potassium, glucose, amino-acids, L-arginine, tetrahydrobiopterin (H4b), vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, their pharmaceutically acceptable salts, and mixtures thereof;
for simultaneous or separate use in the prevention and/or the treatment of a disease selected amongst pigmentosa retinopathy and Stargardt's disease, as stated in claim 9.

Further embodiments of such a kit for use are claimed in claims 10 to 13.

### Detailed Description of the Preferred Embodiments of the Present Invention

The term "vision" or "visual function" as used herein encompasses visual acuity (more especially near and/or far visual acuity) as well as contrast vision, color vision and field of vision (also called visual field herein).

The term "loss of vision" or "degradation of visual function" as used herein includes partial or total loss of vision, and especially a partial or total loss in visual acuity (near and/or far visual acuity) and/or contrast vision and/or color vision and/or field of vision. It can result from a "natural" visual decline (*i.e.,* it appears in the absence of any apparent eye disease or disorder), for example, in an aging animal, and/or from one or several ophthalmic condition(s) (in particular, an eye disorder and/or an eye disease) as disclosed herein.

The terms "treating" and "treatment" mean that an ophthalmologic condition (in particular, an eye disorder and/or an eye disease) is improved (at least partially) and, in particular, that the visual acuity and/or the contrast vision and/or the color vision and/or the field of vision of the treated animal is improved, or that the process of degradation of visual function is stopped.

As used herein, the term "ophthalmologic conditions" (or eye conditions) encompasses ophthalmologic disorders and ophthalmologic diseases involving chorio-retinal and/or optic nerve, resulting in a progressive loss of vision.

The term "ophthalmologic disorders" (or eye disorders) as used herein encompasses changes in vision, in the appearance of the eye or having abnormal sensations in the eye. Eye disorders include optic nerve disorders and chorio-retinal disorders, as well as trauma such as injuries to the eye, and especially disorders resulting in a progressive visual degradation or loss of vision.

As used herein, the term "ophthalmologic diseases" (or eye diseases) means any disease of the eye and, in particular, any disease of the eye resulting in a progressive loss of vision. This terminology encompasses the following diseases of the eye:
- glaucoma neuropathy or glaucoma;
- macular degeneration and, in particular, ARMD, with or without choroidal new vessels;
- diabetic retinopathy
- degenerative chorio retinopathy;
- hereditary dystrophy of the retina and pigmentary epithelium, for example, pigmentosa retinopathy or Stargardt's maculopathy (or Stargardt's disease);
- retinal arterial occlusions (in particular, central retinal artery occlusion);
- retinal vein occlusion (in particular, central retinal vein occlusion or branch retinal vein occlusion);
- uveitis (in particular, anterior uveitis and/or posterior uveitis);
- papillitis;
- ammetropia, for example, myopia (including high myopia), presbyopia or hypermetropia;
- corneal disorders, for example, keratoconjunctivitis (especially keratoconjunctivitis caused by dry eye syndrome, also called keratoconjunctivitis sicca), dry eye syndrome or Fuchs' corneal dystrophy;
- ocular allergy (conjunctivitis);
- age related vision degradation; and
- a natural loss of vision (especially a natural reduction in visual acuity and/or in visual field) and, in particular, physiologic visual decline; and
- night vision decline, and especially hemeralopia.

The term "animal" as used herein includes mammalians, in particular, humans and non human mammalians, and birds.

The term "mammalian" or "mammal" as used herein encompasses any of various warm-blooded vertebrate animals of the class Mammalia, including humans and non human mammals, characterized by a covering of hair on the skin and, in the female, milk-producing mammary glands for nourishing the young.

By "topical administration" it is meant herein an administration which has a local effect. This term includes especially a sub-tenonian administration, or an administration to the eye (especially on intra- or extra- ocular administration).

The administration to the eye can be performed, for example, by applying active principle(s) as disclosed herein (which can be, for example, in the form of an ophtalmologic solution, an ointment or eye drops) to the outside surface of the eye, *i.e.,* by contacting the eye and especially the cornea with said active principle(s).

Alternatively or cumulatively, the administration to the eye can be performed by injecting active principle(s) into the eye and especially into the vitreous (*i.e.,* via intravitreal injection), for example, in the form of an ophtalmologic solution.

Active principles can be administered to the eye (for example, by application to the outside surface of the eye and/or by intraocular injection) using a delivery device which provides a controlled release of active principle(s) on the surface of the eye or into the eye. Said device can be, for example, placed in the lower cul de sac or conjunctival cul-de-sac, below the cornea, or injected into the eye, especially into the vitreous.

By "topical form", it is meant herein a form appropriate for topical administration, and especially a solution (in particular, an ophthalmologic solution), a lotion, drops (in particular, eye drops), a cream or an ointment.

As used herein, the term "pharmaceutically acceptable vehicle" encompasses an ophthalmologically acceptable vehicle (or carrier).

The term "ophthalmologically acceptable vehicle" as used herein means any vehicle that has substantially no long term or permanent detrimental effect on the eye to which it is administered, in particular, any vehicle that can be placed in the eye and that does not cause eye irritation. Opthamologically acceptable vehicles include water (distilled or deionized water), saline solutions, phosphate buffered saline solutions, physiological serum, and other aqueous media.

By "consisting essentially of", it is meant herein that minor ingredients can be added without having a major effect on active principles used in a composition, a medicament, a kit or their use as disclosed herein.

By "several", it is meant herein at least two, *i.e.,* two or more than two (for example, three, four, five, six, seven, eight, nine or ten or more than ten).

The term "adrenergic agent" as used herein encompasses an alpha adrenergic agonist agent, a beta-blocking agent, and mixtures thereof.

As used herein, an "alpha adrenergic agonist agent" is a drug which has effects similar to, or the same as, epinephrine (adrenaline) or which is susceptible to epinephrine, or similar substances, such as biological receptors. This term includes alpha 1 agonists, and alpha 2 agonists. Alpha 1 agonists stimulate phospholipase C activity in a human and an animal body, which results in vasoconstriction and mydriasis (excessive dilation of the pupil). Alpha 2 agonists are able to inhibit adenyl cyclase activity in a human and an animal body and are used notably as antihypertensives, sedatives, to reduce eye's aqueous humor secretions and to facilitate aqueous humor outflow via the uveoscleral route. Examples of alpha 1 agonist include neosynephrine. Examples of alpha 2 agonists include brimonidine, aprachlonidine and clonidine. Others alpha adrenergic agonist agents that can be used and compounds of the present disclosure include methoxamine, methylnorepinephrine, oxymetazoline, phenylephrine, neosynephrine pivalat, beta-methylepinephrine, guanfacine, guanabenz, guanoxabenz, guanethidine, tizanidine, and mixtures thereof.

By "beta-blocking agent" (or beta-adrenergic antagonist agent) it is meant herein a drug which blocks the action of epinephrine (adrenaline) and/or norepinephrine (noradrenaline) in a human and an animal body. These compounds are used notably to lower intraocular tension and/or to reduce eye's aqueous humor secretions. This term encompasses antagonists of the beta 1, beta 2 and beta 3 adrenergic receptors. The beta-blocking agents that can be used, the compositions and the kits of the present disclosure include timolol, sotalol, propranolol, penbutolol, nadolol, metoprolol, labetalol, esmolol, carteolol, bisoprolol, betaxolol, atenolol, acebutolol, levobunolol, metipranolol and mixtures thereof.

In a first aspect, is disclosed a composition, in particular, a pharmaceutical composition (or drug, or medicament), characterized in that it comprises, consists essentially of, or consists of several (*i.e.,* two or more than two) active principles chosen among an angiotensin-converting enzyme inhibitor (also called ACE inhibitor or ACEI herein), folic acid, folate, magnesium, potassium, glucose, amino-acids (for example, a leucine, especially an acetyl-leucine), L-arginine, tetrahydrobiopterin (H4b), vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, a pharmaceutically acceptable salt thereof, and mixtures thereof.

Such a composition as disclosed herein further comprises a pharmaceutically acceptable vehicle, and, in particular, one or several ophthalmologically acceptable vehicle(s). This means that such a composition comprises, consists essentially of or consists of: (i) a combination of active principles as disclosed herein, and one or several pharmaceutically acceptable vehicle(s), especially one or several ophthalmologically acceptable vehicle(s), which can be as disclosed herein.

In one aspect, the composition comprises, consists essentially of, or consists of several active principles chosen among an ACEI, folic acid, folate, magnesium, potassium, glucose, amino-acids (for example, a leucine, especially an acetyl-leucine), L-arginine, H4b, vitamin B6, vitamin B12, vitamin C, w-3 fatty acids, their pharmaceutically acceptable salts, and mixtures thereof.

### Composition 1:

In one aspect, the composition, which is called herein composition 1, comprises, consists essentially of, or consists of:
a) a first active principle, which comprises, consists essentially of, or consists of an ACEI; and
b) a second active principle (or a second active principle mixture), which is chosen among :
   - folic acid, folate, magnesium, potassium, glucose, amino-acids (for example, a leucine, especially an acetyl-leucine), one of their pharmaceutically acceptable salts thereof, and mixtures thereof; and, in particular, which is chosen among:
   - folic acid, folate, glucose, amino-acids (for example, a leucine, especially an acetyl-leucine), one of their pharmaceutically acceptable salts thereof, and mixtures thereof; and
c) optionally, a third active principle, which is chosen among: L-arginine, H4b, vitamin B6, vitamin B12, vitamin C, w-3 fatty acids, anti-inflammatory agents, beta-blocking agents, adrenaline, noradrenaline, alpha adrenergic agonist agents, anti-VEGF agents, one of their pharmaceutically acceptable salts thereof, and mixtures thereof.

In an aspect, the ACEI as used herein is an inhibitor for the enzyme converting angiotensin I to angiotensin II.

In an aspect, the ACEI is more lipophilic in nature than Enalaprilat.

In an aspect, the ACEI comprises, consists essentially of, or consists of Ramipril, Ramiprilat, one of their pharmaceutically acceptable salts, or a mixture thereof.

In another aspect, the ACEI comprises, consists essentially of, or consists of Ramipril.

In an aspect, the second active principle of composition 1 comprises, consists essentially of, or consists of folic acid, folate, one of their pharmaceutically acceptable salts, or a mixture thereof.

In another aspect, the second active principle of composition 1 comprises, consists essentially of, or consists of folic acid.

In an aspect, in composition 1, the first active principle is ramipril and/or ramiprilate and the second active principle is folic acid and/or folate or a mixture of (i) folic acid and/or folate and (ii) magnesium or a pharmaceutically acceptable salt thereof and/or potassium or a pharmaceutically acceptable salt thereof.

In another aspect, composition 1 comprises, consists essentially of or consists of:
- ramipril and folic acid, and
- optionally, magnesium or a pharmaceutically acceptable salt thereof and/or potassium or a pharmaceutically acceptable salt thereof, and
- optionally, a third active principle as disclosed herein and especially a third active principle chosen among L-arginine, H4b, vitamin B6, vitamin B12, vitamin C and w-3 fatty acids.

In another aspect, the second active principle of composition 1 comprises, consists essentially of, or consists of:
- folic acid and/or folate, and
- magnesium (or a pharmaceutically acceptable salt thereof) and/or potassium (or a pharmaceutically acceptable salt thereof), and
- glucose and/or amino-acids (in particular, a leucine as disclosed herein), for example glucose and a leucine as disclosed herein).

Hence, the second active principle of composition 1 can, for example, comprise, consist essentially of, or consist of the following agents: folic acid, magnesium (or a pharmaceutically acceptable salt thereof), potassium (or a pharmaceutically acceptable salt thereof), glucose, and a leucine as disclosed herein, for example, a N-acetyl-leucine and especially the N-acetyl-DL-leucine.

In an aspect, the pharmaceutically acceptable potassium salt as used herein is potassium chloride.

In an aspect, the pharmaceutically acceptable magnesium salt as used herein is magnesium chloride.

In an aspect, by "leucine" it is meant herein acetyl-leucine, for example, N-acetyl-leucine, and more especially acetyl-L-leucine (especially N-acetyl-L-leucine), acetyl-D-leucine (especially N-acetyl-D-leucine) or, more preferably, acetyl-DL-leucine (especially the N-acetyl-DL-leucine). The N-acetyl-DL-leucine is marketed by Pierre Fabre Medicament as an anti-vertigo medicament under the name Tanganil. Alternatively, a DL leucine can also be used as "leucine" according to the present disclosure.

In an aspect, the third active principle of composition 1 comprises or consists of L-arginine or H4b or vitamin B6 or vitamin B12 or vitamin C or a w-3 fatty acid or an anti-inflammatory agent (for example, indomethacin or dexamethasone) or a beta-blocking agent (for example timolol) or adrenaline or noradrenaline or an alpha adrenergic agonist agent (for example, neosynephrine, brimonidine, aprachlonidine or clonidine or mixtures thereof, in particular brimonidine) or an anti-VEGF (for example, bevacizumab, ranibizumab or pegaptanib).

In another aspect, the third active principle of composition 1 comprises, consists essentially of, or consists of a mixture of compounds as set forth in Table 1. This third active principle can be used according to the present disclosure in combination, for example, with (i) Ramipril (or Ramiprilate) and (ii) folic acid (or folate) and/or Magesium (or a magnesium salt) and/or potassium (or a potassium salt and/or a leucine as disclosed herein and/or glucose.

Any w-3 fatty acids can be used according to the present disclosure, and, in particular, a α-linolenic acid (ALA), a eicosapentaenoic acid (EPA), or a docosahexaenoic acid (DHA).

The alpha adrenergic agonist agents that can be used in the composition, a medicament, the kit and the uses as disclosed herein can be selected from the group comprising or consisting of methoxamine, methylnorepinephrine, oxymetazoline, phenylephrine, neosynephrine, in particular, neosynephrine pivalat, beta-methylepinephrine, brimonidine, apraclonidine, clonidine, guanfacine, guanabenz, guanoxabenz, guanethidine, tizanidine, and mixtures thereof. In particular, the alpha adrenergic agonist agent can be neosynephrine, brimonidine, aprachlonidine or clonidine or mixtures thereof, for example brimonidine (Alphagan®), which can be used, for example, in an amount of 0.2% (w/v).

The beta-blocking agents that can be used in the composition, a medicament, the kit and the uses as disclosed herein can be selected from the group comprising or consisting of timolol, sotalol, propranolol, penbutolol, nadolol, metoprolol, labetalol, esmolol, carteolol, bisoprolol, betaxolol, bisoprolol, atenolol, acebutolol, levobunolol, metipranolol and mixtures thereof. In particular, the beta-blocking agent can be timolol, which can be used, for example, in an amount of 0.5% (w/v).

Examples of anti-inflammatory agents that can be used according to the present disclosure are non-steroidal anti-inflammatory agents or steroidal anti-inflammatory agents, in particular, corticosteroids, or mixtures thereof.

Non-steroidal anti-inflammatory drugs can be selected, for example, among aspirin, arylalkanoic acids, in particular, bromfenac, indometacin, oxameticin, 2-arylpropionic acids, in particular, fenbufen, pirprofen, ketoprofen, ibuprofen, oxaprozin, and ketorolac, femamic acids, pyrazolidine derivatives, in particular, clofezonem kebuzone and phenazone, ocicams, in particular, droxicam and meloxicam, and COX-2 inhibitors, in particular, celecoxib and rofecoxib. In particular, the non steroidal anti-inflammatory agent can be indomethacin (Indocollyre ®), which can be used, for example, in an amount of 0.1 % (w/v).

Examples of corticosteroids that can be used in the composition, a medicament, the kit and the uses disclosed herein can be selected among cortisone, hydrocortisone, deltacortisone or prednisolone, prednisone, deltahydrocortisone or prednisolone, methylprednisolone or medrocortisone, fluorohydrocortisone or fluorocortisone, fluoromethylprednisolone or dexamethazone, fluoromethyldeltahydrocortisone or betamethazone and paramethazone. In particular, the corticosteroid can be dexamethasone (Tobradex®), which can be used, for example, in an amount of 0.1% (w/v).

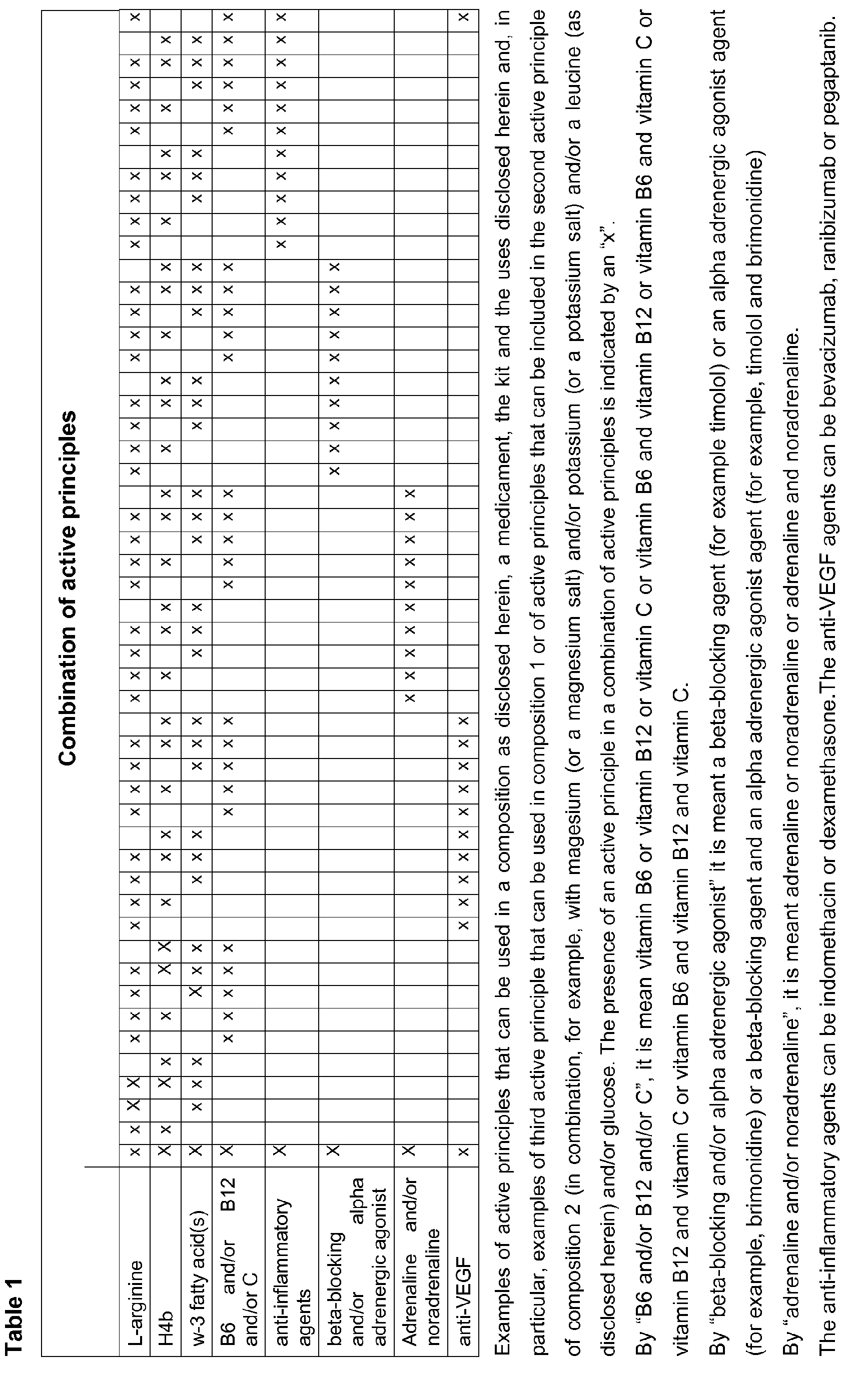

Anti-VEGF agents that can be used in the uses, the compositions and the kits of disclosed herein can be selected among bevacizumab (Avastin®), ranibizumab (Lucentis®), pegaptanib (Macugen®), and mixtures thereof. In particular, the Anti-VEGF agent can be bevacizumab or ranibizumab.

### Composition 2:

In another aspect, the composition, which is called herein composition 2, comprises, consists essentially of, or consists of:
a) a first active principle (or a first active principle mixture), which is chosen among folic acid, folate, one of their pharmaceutically acceptable salts, and mixtures thereof; and
b) a second active principle (or a second active principle mixture), which is chosen among: magnesium, potassium, glucose, amino-acids (for example, leucine, especially acetyl-leucine), L-arginine, H4b, vitamin B6, vitamin B12, vitamin C, w-3 fatty acids, one of their pharmaceutically acceptable salts, and mixtures thereof; and
c) optionally, a third active principle, which is chosen among: anti-inflammatory agents, beta-blocking agents, adrenaline, noradrenaline, alpha adrenergic agonist agents, anti-VEGF agents, one of their pharmaceutically acceptable salts, and mixtures thereof.

In an aspect, the first active principle of composition 2 comprises, consists essentially of, or consists of folic acid and/or folate.

In an aspect, the second active principle of composition 2 comprises, consists essentially of, or consists of:
- magnesium (or a pharmaceutically acceptable salt thereof) and/or potassium (or a pharmaceutically acceptable salt thereof) and/or glucose and/or amino-acids (for example, leucine, especially acetyl-leucine) and/or L-arginine, and
- optionally, an active principle chosen from H4b, vitamin B6, vitamin B12, vitamin C, w-3 fatty acids, one of their pharmaceutically acceptable salts thereof, and mixtures thereof.

In another aspect, in composition 2:
- the first active principle comprises, consists essentially of, or consists of folic acid and/or folate; and
- the second active principle comprises, consists essentially of, or consists of:
   (i) magnesium (or a pharmaceutically acceptable salt thereof), potassium (or a pharmaceutically acceptable salt thereof), glucose, and a leucine as disclosed herein, for example, a N-acetyl-leucine and especially the N-acetyl-DL-leucine; or
   (i) magnesium (or a pharmaceutically acceptable salt thereof), potassium (or a pharmaceutically acceptable salt thereof), glucose, a leucine as disclosed herein (for example, a N-acetyl-leucine and especially the N-acetyl-DL-leucine) and L-arginine,
- the third active principle being or not being present in said composition. In another aspect, in composition 2:
- the first active principle comprises, consists essentially of, or consists of folic acid and/or folate; and
- the second active principle comprises, consists essentially of, or consists of:
   (i) magnesium (or a pharmaceutically acceptable salt thereof) and/or potassium (or a pharmaceutically acceptable salt thereof) and/or a leucine as disclosed herein and/or glucose; and
   (ii) a mixture of compounds as set forth in Table 1;
- the third active principle being or not being present in said composition.

### Composition 3:

In another aspect, the composition, which is called herein composition 3, comprises, consists essentially of, or consists of:
a) a first active principle (or a first active principle mixture), which is chosen among glucose, one of its pharmaceutically acceptable salts, and mixtures thereof; and
b) a second active principle (or a second active principle mixture), which is chosen among: magnesium, potassium, one of their pharmaceutically acceptable salts, and mixtures thereof, and
c) a third active principle chosen among amino-acids, for example, a third active principle which comprises or consists of a leucine or a pharmaceutically acceptable salts, for example, a N-acetyl-leucine and especially the N-acetyl-DL-leucine.

In an aspect, composition 3 comprises, consists essentially of, or consists of:
- glucose, as first active principle;
- magnesium (or one of its pharmaceutically acceptable salts) and potassium, (or one of its pharmaceutically acceptable salts) as second active principle; and
- a leucine as disclosed herein, for example, a N-acetyl-leucine and especially the N-acetyl-DL-leucine, as third active principle.

In an aspect, the third active principle of composition 3 further comprises L-arginine.

In an aspect, the composition (for example, composition 1, 2 or 3) is a cellprotector and/or a neuroprotector and/or a retinoprotector.

In an embodiment, a composition or a medicament as disclosed herein or as claimed is suitable for preventing, slowing down or interrupting the visual function degradation process or even reversing its course in an animal (especially a human or non human mammal) and, in particular, in an aging animal and/or in an animal with an eye condition (especially an eye disease or disorder).

Hence, in an embodiment, said composition is suitable for maintaining or improving visual acuity and/or field of vision in an animal (especially a human or non human mammal) and, in particular, in an aging animal and/or in an animal with an eye condition (especially an eye disease or disorder). Said composition can also be applied to a normal subject (*i.e.* to an animal which does not have any apparent eye disease or disorder), for maintaining eye vision (*i.e.,* for preventing a loss of vision) or even for improving visual acuity and/or visual field.

The composition or medicament as disclosed herein or as claimed can be in a form appropriate for (and, in particular, can comprise a vehicle appropriate for) enteral (for example, oral), parenteral (for example, intravenous, intramuscular or subcutaneous), transdermal, or local or topical administration, and especially topical administration to the eye. Forms appropriate for administration to the eye include the ones which are appropriate for application to the outside surface of the eye, for intraocular administration (or injection), especially intravitreal injection and/or for sub-tenonian administration.

In one aspect, the composition (for example, composition 1, 2 or 3 as disclosed herein) is in the form of a tablet, a solution (for example, an ophthalmic solution), a lotion, drops (for example, eye drops), a cream or an ointment.

In another aspect, the composition (for example, composition 1, 2 or 3 as disclosed herein) is in the form of an ophthalmic solution, an eye ointment or eye drops, such a composition being obtainable, for example, by diluting the active principles in an opthamologically acceptable vehicle, for example, in a physiological serum.

The composition or kit for use of the invention can be in the form stated in claim 4, 5 or 11.

In an embodiment, the composition disclosed herein (for example, composition 1 as disclosed herein) is an ophthalmic solution or eye drops which comprises, consists essentially of, or consists of ramipril and folic acid.

In yet another embodiment, the composition disclosed herein (for example, composition 2 as disclosed herein) is an ophthalmic solution or eye drops which comprises, consists essentially of, or consists of folic acid, magnesium (or a pharmaceutically acceptable salt thereof), potassium (or a pharmaceutically acceptable salt thereof), glucose, a leucine as disclosed herein (for example, the N-acetyl-DL-leucine), and optionally L-arginine.

In another embodiment, the composition (for example, composition 3 as disclosed herein) is an ophthalmic solution or eye drops, which comprises, consists essentially of, or consists of glucose, magnesium (or one of its pharmaceutically acceptable salts), potassium (or one of its pharmaceutically acceptable salts) and a leucine as disclosed herein (for example, the N-acetyl-DL-leucine), and optionally L-arginine.

In another aspect, is disclosed a composition as disclosed herein, for use as a medicament. In particular, this composition can be used as a cellprotector, a neuroprotector and/or a retinoprotector medicament.

According to an aspect of the present disclosure, the composition disclosed herein is for use in the prevention or the treatment of any one or several disease(s) or disorder(s) chosen from: ophthalmologic conditions (especially ophthalmologic diseases and disorders as disclosed herein), cancers (for example, carcinoma, and more especially adeno-carcinoma), arterial hypertension, hyperlipemia, coronary heart disease, atherosclerosis, diabete, neurodegenerative conditions (for example, multiple sclerosis, Alzheimer disease and/or Parkinson disease), rheumatism, general inflammatory and immune conditions and infections (for example, viral and especially HIV infection, bacterial infection and/or parasitic infection).

According to an aspect of the present disclosure, the composition disclosed herein is for use in the prevention or the treatment of one or several ophthalmologic condition(s) as disclosed herein and, in particular, of one or several ophthalmologic condition(s) chosen from: glaucoma neuropathy, glaucoma, myopia, presbyopia, ammetropia, hereditary dystrophy of the retina and pigmentary epithelium, for example, pigmentosa retinopathy or Stargardt's disease, ARMD, diabetic retinopathy and keratoconjunctivitis caused by dry eye syndrome.

According to the invention, the ophthalmologic condition is as stated in claim 1 and 9.

According to an aspect of the present disclosure, the ophthalmologic condition is an hereditary dystrophy of the retina and pigmentary epithelium. According to the invention, the ophthalmologic condition is pigmentosa retinopathy or Stargardt's disease, and the treated patients are, according to a particular aspect, under the age of forty, preferably under the age of thirty, and more preferably under the age of twenty, for example, from 6 to 30 or from 6 to 20 years of age.

In an embodiment, the composition, a medicament, the kit and the uses disclosed herein or according to the invention are intended for or used to prevent, slow down, stop or even reverse (at least partially) a loss of vision and especially a loss of visual acuity and/or field of vision, said loss of vision resulting, for example, from a natural visual decline and/or from an ophthalmologic disorder or disease as disclosed herein.

Hence, in an embodiment, the composition, a medicament, the kit and their uses are intended for or used for maintaining or improving (*i.e.,* increasing) eye vision (in particular, visual acuity and/or visual field), for example, in a normal subject (*i.e.* in an animal which does not have any apparent eye disease or disorder) and/or in an aging animal (especially an aging human or non human mammal) and/or in an animal having a ophthalmologic condition as disclosed herein.

The composition, a medicament, the kit and uses can, in particular, be intended for or used for maintaining or improving vision of one or both eyes, and more particularly for improving distance vision and/or near vision of one or both eyes in a normal animal and/or in an aging animal
(especially an aging human or non human mammal) and/or in an animal having a ophthalmologic condition as disclosed herein.

In an aspect, is disclosed a cellprotective, neuroprotective and retinoprotective composition comprising or consisting of angiotensin converting enzyme inhibitor (for example, Ramipril or Ramiprilate) associated with folic acid, with or without magnesium, with or without potassium, with or without vitamin B6, with or without vitamin B12, with or without vitamin C, with or without L-arginine, with or without w-3 fatty acids, with or without H4B, with or without glucose, and with or without amino-acids (for example, with or without a leucine, especially an acetyl-leucine, and more especially the N-acetyl-DL-leucine), which composition is sufficiently stable not only to interrupt the visual function degradation process in an animal (especially a patient) suffering from an ophthalmologic condition (especially a ophthalmologic disorder or disease) as disclosed herein, but also to reverse or regress that process.

In another aspect, the disclosure concerns the use of the active principles described herein for the manufacture of a composition, a medicament or a kit (as disclosed hereinafter) intended for use in inducing an improvement in visual acuity and field of vision in a treated animal (especially a human or non human mammal).

In another aspect, the disclosure also relates to a kit which comprises or consists of :
- several (at least two) active principles chosen among an ACEI, folic acid, folate, magnesium, potassium, glucose, amino-acids (especially a leucine and, in particular, an acetyl-leucine), L-arginine, H4b, vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, their pharmaceutically acceptable salts thereof and mixtures thereof; and
- optionally, instructions for using said kit,
wherein said active principles are associated in the same composition or wherein at least two of these active principles are in separate compositions.

According to another aspect, the kit comprises or consists of:
(i) a first active principle; and
(ii) a second active principle; and
(iii) optionally, a third active principle; and
(iv) optionally, instructions for using said kit (for example, in a use as disclosed herein),
wherein the first active principle, the second active principle and - if present - the third active principle are as disclosed herein for composition 1, 2 or 3 and are associated in the same composition or wherein at least two of these active principles are in separate compositions.

The term "associated in the same composition" (or "present in the same composition") means that the active principles present in the kit and, in particular, the first active principle, the second active principle and - if present - the third active principle as disclosed herein for composition 1, 2 or 3 are in the form of one single composition, *i.e.,* that the kit comprises a composition as disclosed herein (for example, composition 1, 2 or 3).

Alternatively, when "at least two of these active principles are in separate compositions", this means that the kit comprises at least two or three separate compositions, each of these compositions comprising, consisting essentially of, or consisting of one or several active principle(s) (and a pharmaceutically acceptable vehicle, especially an ophthalmologically acceptable vehicle). For example, a kit which comprises a first active principle, a second active principle and optionally a third active principle as disclosed herein for composition 1, 2 or 3:
- may comprise only two separate compositions when:
   (i) the third active principle is not present (in this case, one composition comprises the first active principle and the other composition comprises the second active principle); or
   (ii) the third active principle is present but either the first, the second or the third active principle is separated from the two other active principles in a separate composition; and
- may comprises three separate compositions when the third active principle is present and when each of the three active principles is present in a separate composition (*i.e.,* each of these three compositions comprises, consists essentially of, or consists of one of the three active principles, and a pharmaceutically acceptable vehicle, especially an ophthalmologically acceptable vehicle).

Or course, when the first active principle, the second active principle or the third active principle which is used in the kit or the uses disclosed herein comprises a mixture of several active principles, these active principles can be present in the same composition or alternatively at least two of these active principles can be in separate compositions.

The at least two or three separate compositions which can be present in the kit can be administered either simultaneously or not (in any order) to an animal (especially an aging human or non human mammal), for example, to carry out a use as disclosed herein.

In an aspect, the active principles present in the kit and, in particular, the first, the second and - if present - the third active principle present in the kit are associated with pharmaceutically acceptable vehicles allowing their administration, in different forms, in particular: enteral forms especially oral forms, parenteral forms, for example, intravenous forms or intramuscular forms, transdermal forms, and topical forms, in particular, topical forms appropriate for administration to the eye, including the topical forms appropriate for application to the outside surface of the eye, for intraocular or intravitreal injection and/or for sub-tenonian administration (for example, eye or ophthalmic solutions or eye drops). Clearly, the dosage of each of the active principles present in a composition, a medicament or a kit according to the present disclosure can vary from one patient to another and fine tuning can be left to the clinician. When a composition, a medicament or a kit according to the present disclosure are intended for treating of preventing a loss of vision and especially for treating of preventing eye conditions as disclosed herein, there is clear preference for topic forms of administration, in particular, eye or ophthalmic solutions or eye drops. In other words, in the preferred administration forms of a composition or medicament or of the active principles present in the kit disclosed herein, the combined active principles used are associated with pharmaceutical vehicles that allow their clinical application in the form of eye lotions, eye ointment, eye solutions, or eye drops, preferably eye solutions or eye drops.

In an aspect, at least two of the active principles of the kit are in separate compositions, and at least one of these separate compositions is formulated in a topical form appropriate for administration to the eye, for example, a form appropriate for contacting the eye with active principles as disclosed herein (especially eye drops or an eye solution) and/or for intraocular or intravitreal injection and/or is used for administration to the eye (for example, by contacting the eye with active principles as disclosed herein or via intraocular or intravitreal injection). In this case, the other separate composition(s) can be formulated either in a topical form appropriate for administration to the eye (for example, eye drops or an eye solution) or in another administration form, for example, in an oral form.

In a further aspect, is disclosed a composition for use for preventing and/or treating one or several disease(s) or disorder(s) (in particular, one or several eye condition(s) as disclosed herein) in an animal (especially a human or non human mammal) in need thereof, encompassing administration to said animal of at least two active principles as defined herein and, in particular, a first active principle, a second active principle and optionally a third active principle as defined herein for composition 1, 2 or 3 (or composition(s) or medicament(s) comprising them), wherein said active principles are administered separately (*i.e.,* in separate compositions) or not separately (for example, in the same composition), and wherein said disease(s) or disorder(s) is(are), for example, as defined herein.

In another aspect, is disclosed a composition for use for maintaining or improving vision and, in particular, the visual acuity and/or the field of vision in an animal (especially a human or non human mammal) in need thereof, encompassing administration to said animal of at least two active principles as defined herein and, in particular, a first active principle, a second active principle and optionally a third active principle as disclosed herein for composition 1, 2 or 3 (or composition(s) or medicament(s) comprising them), wherein said active principles are administered separately (*i.e.,* in separate compositions) or not separately.

By "administered separately or not", it is meant herein administered in the form of separate compositions or not. Hence, when they are administered "separately", this means that at least two or three separate compositions are used: in particular, when a first active principle, a second active principle and optionally a third active principle as disclosed herein are administered, at least two separate compositions are used when there is no third active principle and at least three separate compositions are used when a third active principle is used. By "not administered separately", it is meant herein that at least two of these active principles are in separate compositions (as disclosed herein for the kit). These separate compositions can be administered simultaneously or not to the animal.

The uses disclosed herein can be performed using the kit disclosed herein.

In an aspect, the composition(s) comprising active principles (for example, the first active principle and/or the second active principle and/or the third active principle as disclosed herein for composition 1, 2 or 3) which is(are) administered according to a use disclosed herein is(are) cellprotector, neuroprotector and/or retinoprotector composition(s).

In an embodiment, by "administering" it is meant herein applying active principles (for example, the first, the second and/or the third active principle as disclosed herein) and, in particular, the composition disclosed herein to the outside surface of one eye or both eyes of an animal (especially a human or non human mammal), and, in particular, contacting the surface of one eye or of both eyes of an animal with said active principles or with a composition as disclosed herein. The administered active principles or composition can be, for example, in the form of an ophtalmologic solution, eyedrops or an ointment.

Alternatively or cumulatively, in an embodiment, by "administering" it is meant herein injecting in one eye or both eyes, especially injecting into the vitreous of one eye or of both eyes of an animal (especially a human or non human mammal), active principle(s) (in particular, the first, the second and/or the third active principle as disclosed herein), which active principle(s) can be, for example, in the form of an ophtalmologic solution.

The active principles and, in particular, the first active principle and/or the second active principle and/or the third active principle (or composition(s) comprising them) can be administered by the same route (for example, the topical route as disclosed herein) or via different routes, which routes can be chosen independently, for example, from the administration routes disclosed herein.

At least one of these active principles can be in the form of an ophthalmic solution or eye drops or ointment.

In one aspect of the uses disclosed herein (especially for maintaining or improving vision according to the present disclosure), the treated animal (especially a human or non human mammal) has one or several ophthalmologic condition(s) as defined herein and/or is aged.

In an embodiment, a composition, a medicament, a kit or a use according to the present disclosure is intended for or applied to a human being (or patient). In this embodiment, by "aged" or "aging" it is meant, for example, above the age of 50, above the age of 60 or above the age of 70.

Effective amounts and/or pharmaceutically acceptable amounts (especially physiologically or ophtalmologically acceptable amounts) of active principles, and especially of the first active principle, the second active principle and - if any - the third active principle are used.

As a way of illustration, an active principle is usually administered to a human or an animal in order to prevent or treat an eye condition in a concentration ranging from 0.001 to 15% (w/v), preferably from 0.05 to 10% (w/v), and more preferably from 0.1 to 3% (w/v). In particular, an active principle in the form of eye drops can be used, for example, in a concentration ranging from 0.1 to 5 % and more preferably from 0.5 to 3%, for example, in a concentration of 0.5%, 1 % or 2%.

Especially Ramipril or Ramiprilate can be administered to a human:
- topically (for example, in the form of eye drops) in a concentration of 0.5 to 5 % or 0.5 to 3%, for example, in a concentration of 0.5%, 1% or 2%; and/or
- orally in an amount of 0.5 to 5 mg/day, preferably 1 to 2 mg/day, for example, 1.25 mg/day; and

Folic acid or folate can be administered to a human,
- topically (for example, in the form of eye drops) in a concentration of 0.5 to 5 % or 0.5 to 3%, for example, in a concentration of 0.5%, 1% or 2%; and/or
- orally, in an amount of 2 to 8 mg/day, preferably 4 to 6 mg/day, for example, 5 mg/day.

The compositions or medicaments that contain the active principles as defined herein may be administered to a mammalian eye as often as necessary to obtain an improvement of the disorder or disease (and especially of the ophthalmic condition). Those skilled in the art will recognize that the frequency of administration and duration of treatment depends on the precise nature of the active principles and its concentration in the composition, and various factors such as the type and severity of the disorder or disease, the age and weight of the animal, the animal's general physical condition and the cause of the disorder or disease. Within these guidelines, it is contemplated that the ophthalmic composition (preferably ophthalmic solutions or eye drops) disclosed herein will be administered topically to the mammalian eye and, in particular, dropped into the eye and/or injected into the mammalian eye approximately once, twice or three times daily.

The duration of treatment administered in accordance with the present disclosure may range, for example, from a few weeks (at least one week) to a few months (at least one month), in particular, from 1 week to 6 months, preferably at least 2 weeks and less than 4 months and more preferably at least 3 weeks and less than 3 months. However, a prolonged treatment may be required. In particular, the treatment may last for one or several years or even for life, for example, in case of recurrence of the disorder(s) or disease(s) and especially of an ophthalmic condition.

Of course, one of several additional active principle(s), and especially one of several additional compounds for treating eye disorders and/or diseases may be used in the uses disclosed herein or may be present in a composition for use, a medicament or a kit for use according to the present disclosure, provided that they do not interact with the first and second active principles and - if present - with the third active principle, to provide adverse side effects.

The invention will be illustrated further by the description of the following clinical examples.

### EXAMPLES - CLINICAL OBSERVATIONS

In the case of the clinical cases disclosed below, the active principles, in particular, Ramipril and folic acid were administered continuously (*i.e.,* at least once a day), in an oral form of 1.25 mg per day for Ramipril and 5mg per day for folic acid or in a topical form.

With respect to the clinical observations disclosed below, it is notable that equivalent observations were made in "normal" patients, which do not present any ocular pathology (in particular, aging patients above the age of 60), to whom the medicament was administered; these patients noticed an improvement of their vision, in particular, aging individuals regained the mean sensitivity that they experienced when they were much younger. Examples below, which do not relate to pigmentosa retinopathy or Stargardt's disease are provided as comparative examples only.
***GLAUCOMA AND GLAUCOMATOUS NEUROPATHY (comparative example):*** the administration of the combination of Ramipril and folic acid in oral or topic form induces improvement in the visual function (visual acuity or visual field) and decreased intraocular pressure in 18 individuals.
***MYOPIA (comparative example):*** 3 patients were tested. The patient's vision was tested before and after the first eye-drop formulation was administered. 2 hours later, the patient's unaided distance vision improved. Wearing distance - corrected glasses, the patient vision improved.
***PRESBYOPIA (comparative example):*** 5 patients were tested. The patient's near vision was tested before treatment. 2 hours after this treatment the patient's near vision increased.
***AMMETROPIA (comparative example):*** improvement of near and distance vision 2 hours after treatment.
***HEREDITARY DYSTROPHY OF THE RETINA AND PIGMENTARY EPITHELIUM:***
***1- Retinitis pigmentosa:*** this disorder corresponds to a dystrophy in the cones and rods. It is characterized by the appearance of night blindness in infancy or adolescence, progressive contractions of the peripheral field of vision and results in a substantial loss in visual acuity or even blindness by adulthood. Four patients were treated orally or topically and their visual acuity improved.
***2- Stargardt's disease:***
   Two patients were treated visual. Acuity improved from 2 to 4/10.
***AGE RELATED MACULAR DEGENERATION (ARMD) (comparative example):*** 12 patients were tested. The majority of patients presenting an age-related macular degeneration with or without sub-retinal neovessels treated with the combination Ramipril and folic-acid experienced improved visual acuity.
***DIABETIC RETINOPATHY (comparative example):*** All 10 patients were treated with the combination Ramipril and folic acid improved their vision and ocular fundus appearance.
***KERATOCONJUNCTIVITIS BY DRY EYE (comparative example):*** 2 patients received this treatment and their conditions were improved.

The term "visual function" as used herein should be understood to refer more particularly to visual acuity or the field of vision or, as is preferable, to both at once. The disclosure thus concerns, in an aspect, a cellprotective, neuroprotective and retinoprotective ophthalmologic drug that can interrupt the process of degradation of visual function and even reverse its course.

According to a particular aspect, the combination of Ramipril and folic acid can also be associated, for example, with one or several compounds chosen among vitamin B12, vitamin B6, Vitamin C, H4B, L-arginine, w-3 fatty acids, magnesium, potassium, glucose, and amino-acids (for example, leucine and especially acetyl-leucine).
***CANCER (comparative example):*** an eighty old woman presented with an adeno-carcinoma (cancer) in the tongue. She received the same treatment (*i.e.,* ramipril and folic acid), which was administered by the oral route and after four months of daily treatment, the volume of the tumour was decreased by about 30%.

It appears that the beneficial effect achieved by the folic acid and Ramipril combination may be explained by the increase of nitric oxide (NO) bioavailability in a cell. This is only a hypothesis and must be further explored.

The increase of NO bioavailability improves endothelial function, significantly reduces endothelial dysfunction, acts against oxidative stress, significantly reduces inflammation, has antithrombotic activity and inhibits blood clot formation.

Ramipril as an ACEI or Ramiprilat reduces degradation of bradykinin stimulating NO reduction. Long term bradykinin stimulation enhances the release of NO, causing vasodilatation. Ramipril (or Ramiprilat) drastically reduces superoxide production and oxidative stress. But NO formation is critically dependant on the availability of the cofactor H4B which stimulates the conversion of L-arginine to L-citrulline and NO by NOS (NO synthase). Folic acid or folate (vitamin B9) stimulates endogenous H4B regeneration, a cofactor necessary for eNO synthesis, inhibits intracellular superoxide generation, and thus enhances the half-life of NO.

Impaired endothelial NO (eNO) activity is an early marker for cardiovascular disease. Most risk factors for atherosclerosis are associated with impaired endothelium dependent vasodilatation due to reduced NO production. Folate not only reduces plasma homocysteine levels but also enhances eNO synthesis and shows anti-inflammatory actions. It stimulates endogenous H4B regeneration, a cofactor necessary for eNO synthesis, inhibits intracellular superoxide generation, and this enhances the half-life of NO. Vitamin C augments eNO synthesis by increasing intracellular H4B. The ability of folate to augment eNO generation is independent of its capacity to lower plasma homocystein levels.

In conclusion, it is proposed that folic acid, vitamin c, w-3 fatty acids, H4B, L arginine and magnesium, augment the activity of Ramipril and thus may act in synergy with Ramipril by enhancing NO production. This would explain why this association is more efficient to prevent or to treat some ocular pathologies and presents as a more cellprotective, neuro and retinoprotective than these drugs used alone.

Hence, the common mechanism by which Ramipril, folic acid, vitamin c, w-3 fatty acids, H4B, L arginine and magnesium, bring about their beneficial actions in various vascular and ocular diseases is thought to be by enhancing eNO production.

In view of the clinical observations, the combination of Ramipril and folic acid and, optionally vitamin B6, vitamin B12, vitamin C, w-3 fatty acids, H4B, L-arginine, potassium, magnesium, glucose and/or and amino-acids (for example, leucine and especially acetyl-leucine) could be judicious for a novel approach in the prevention and management of various conditions such as eye conditions (as disclosed herein) arterial hypertension, hyperlipidemia, coronary heart disease, atherosclerosis, diabete, neurodegenerative conditions (multiple sclerosis, Alzheimer disease, Parkinson disease, etc...), rheumatism, general inflammatory and immune conditions, infections (viral, bacterial and/or parasitic infections), especially HIV infections, and cancer.

## Claims

1. A composition for use in the prevention and/or the treatment of a disease selected amongst pigmentosa retinopathy and Stargardt's disease, **characterized in that** it comprises:
a. a first active principle, which consists of an angiotensin-converting enzyme inhibitor (ACEI) selected from the group consisting of: Ramipril, Ramiprilate, and one of their pharmaceutically acceptable salts, and
b. a second active principle that is selected from the group consisting of: folic acid, folate, and one of their pharmaceutically acceptable salts, and
c. optionally, at least a further active principle chosen among: magnesium, potassium, glucose, amino-acids, L-arginine, tetrahydrobiopterin (H4b), vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, their pharmaceutically acceptable salts, and mixtures thereof.

2. The composition for use according to claim 1, wherein the first active principle is ramipril and the second active principle is folic acid, the composition further comprising magnesium or a pharmaceutically acceptable salt thereof and/or potassium or a pharmaceutically acceptable salt thereof.

3. The composition for use according to claim 1 or 2, wherein the leucine is an acetyl-leucine and, in particular, an N-acetyl-DL-leucine.

4. The composition for use according to any one of claims 1 to 3, which is in a form appropriate for enteral, especially oral, parenteral, especially intravenous, intramuscular or subcutaneous, transdermal, or topical administration, especially for topical administration to the eye, including intravitreal injection, or for sub-tenonian administration.

5. The composition for use according to any one of claims 1 to 4, which is in the form of a tablet, a solution, a lotion, drops, a cream or an ointment.

6. The composition for use according to any one of claim 1 to 5, which is in the form of an ophthalmic solution or eye drops.

7. The composition for use according to any one of claims 1 to 6, wherein
- Ramipril or Ramiprilate is administered topically in a concentration of 0.5 to 5 % or 0.5 to 3%, for example, 0.5%, 1% or 2%, and/or orally, in an amount of 0.5 to 5 mg/day, preferably 1 to 2 mg/day, for example, 1.25 mg/day to a human; and/or
- folic acid or folate is administered topically in a concentration of 0.5 to 5 % or 0.5 to 3%, for example, 0.5%, 1% or 2%, and/or orally, in an amount of 2 to 8 mg/day, preferably 4 to 6 mg/day, for example, 5 mg/day to a human.

8. The composition for use according to claim 7, wherein Ramipril or Ramiprilate and/or folic acid or folate is(are) administered topically in the form of eye drops.

9. A kit comprising:
a. a first active principle, which consists of an angiotensin-converting enzyme inhibitor (ACEI) selected from the group consisting of: Ramipril, Ramiprilate, and one of their pharmaceutically acceptable salts, and
b. a second active principle that is selected from the group consisting of: folic acid, folate, and
c. optionally, at least a further active principle chosen among: magnesium, potassium, glucose, amino-acids, L-arginine, tetrahydrobiopterin (H4b), vitamin B6, vitamin B12, vitamin C, w-3 fatty acids, anti-inflammatory agents, beta-blocking agents, adrenaline, noradrenaline, alpha adrenergic agonist agents, anti-vascular endothelial growth factor (anti-VEGF) agents, their pharmaceutically acceptable salts, and mixtures thereof;
for simultaneous or separate use in the prevention and/or the treatment of a disease selected amongst pigmentosa retinopathy and Stargardt's disease.

10. Kit for use according to claim 9, wherein the first active principle and/or the second active principle and/or the third active principle if any are administered by the same route or by different routes.

11. Kit for use according to any one of claims 9 to 10, wherein at least one of the active principles is in the form of an ophthalmic solution or eye drops or ointment.

12. Kit for use according to any one of claims 9 to 11, wherein
- Ramipril or Ramiprilate is administered topically in a concentration of 0.5 to 5 % or 0.5 to 3%, for example, 0.5%, 1% or 2%, and/or orally, in an amount of 0.5 to 5 mg/day, preferably 1 to 2 mg/day, for example, 1.25 mg/day to a human; and/or
- folic acid or folate is administered topically in a concentration of 0.5 to 5 % or 0.5 to 3%, for example, 0.5%, 1% or 2%, and/or orally, in an amount of 2 to 8 mg/day, preferably 4 to 6 mg/day, for example, 5 mg/day to a human.

13. Kit for use according to claim 12, wherein Ramipril or Ramiprilate and/or folic acid or folate is(are) administered topically in the form of eye drops.

## Patentansprüche

1. Zusammensetzung zur Verwendung bei der Vorbeugung und / oder der Behandlung einer Krankheit, die ausgewählt ist aus Retinitis pigmentosa und der Stargardt-Krankheit, **dadurch gekennzeichnet, dass** sie umfasst:
a. einen ersten wirksamen Bestandteil, der aus einem Angiotensin-Konversionsenzym-Hemmer (ACEI) besteht, der aus der Gruppe ausgewählt ist, bestehend aus: Ramipril, Ramiprilat und einem ihrer pharmazeutisch akzeptablen Salze davon, und
b. einen zweiten wirksamen Bestandteil, der ausgewählt ist aus der Gruppe, bestehend aus: Folsäure, Folat und einem ihrer pharmazeutisch akzeptablen Salze, und;
c. optional wenigstens einen weiteren wirksamen Bestandteil, der ausgewählt ist aus: Magnesium, Kalium, Glukose, Aminosäuren, L-Arginin, Tetrahydrobiopterin (H4b), Vitamin B6, Vitamin B12, Vitamin C, w-3-Fettsäuren, entzündungshemmenden Wirkstoffen, Betablocker-Wirkstoffen, Adrenalin, Noradrenalin, alpha-adrenergischen, agonistischen Wirkstoffen, antivaskulären, endothelialen Wachstumsfaktor-(anti-VEGF)-Wirkstoffen, deren pharmazeutisch akzeptablen Salzen und den Mischungen davon.

2. Zusammensetzung zur Verwendung gemäß Anspruch 1, wobei der erste wirksame Bestandteil Ramipril ist und der zweite wirksame Bestandteil Folsäure ist, wobei die Zusammensetzung weiterhin Magnesium oder ein pharmazeutisch akzeptables Salz davon und / oder Kalium oder ein pharmazeutisch akzeptables Salz davon umfasst.

3. Zusammensetzung zur Verwendung gemäß Anspruch 1 oder 2, wobei das Leucin ein Acetyl-Leucin und insbesondere ein N-Acetyl-DL-Leucin ist.

4. Zusammensetzung zur Verwendung gemäß irgendeinem der Ansprüche 1 bis 3, das in einer zur enteralen, insbesondere oralen, parenteralen, insbesondere intravenösen, intramuskulären oder subkutanen, transdermalen oder topischen Verabreichung, insbesondere zur topischen Verabreichung am Auge, unter Einschluss einer intravitrealen Injektion, oder zur subtenösen Verabreichung geeignet ist.

5. Zusammensetzung zur Verwendung gemäß irgendeinem der Ansprüche 1 bis 4, die die Form einer Tablette, einer Lösung, einer Lotion, von Tropfen, einer Creme oder einer Salbe aufweist.

6. Zusammensetzung zur Verwendung gemäß irgendeinem der Ansprüche 1 bis 5, die die Form einer ophthalmologischen Lösung oder von Augentropfen aufweist.

7. Zusammensetzung zur Verwendung gemäß irgendeinem der Ansprüche 1 bis 6, wobei
- Ramipril oder Ramiprilat einem Menschen topisch in einer Konzentration von 0,5 bis 5 % oder 0,5 bis 3 %, z. B., 0,5 %, 1 % oder 2 % und / oder oral in einer Menge von 0,5 bis 5 mg / Tag, bevorzugt 1 oder 2 mg / Tag, z. B. 1,25 mg / Tag verabreicht wird; und / oder
- Folsäure oder Folat einem Menschen topisch in einer Konzentration von 0,5 bis 5 % oder 0,5 bis 3 %, z. B. 0,5 %, 1 % oder 2 % und / oder oral in einer Menge von 2 bis 8 mg / Tag, bevorzugt 4 bis 6 mg / Tag, z. B. 5 mg / Tag verabreicht wird.

8. Zusammensetzung zur Verwendung gemäß Anspruch 7, wobei Ramipril oder Ramiprilat und / oder Folsäure oder Folat topisch in Form von Augentropfen verabreich ist / wird.

9. Kit, umfassend:
a. einen ersten wirksamen Bestandteil, der aus einem Angiotensin-Konversionsenzym-Hemmer (ACEI) besteht, der aus der Gruppe ausgewählt ist, bestehend aus: Ramipril, Ramiprilat und einem ihrer pharmazeutisch akzeptablen Salze, und
b. einen ersten wirksamen Bestandteil, der aus der Gruppe ausgewählt ist, bestehend aus: Folsäure, Folat und
c. optional wenigstens einem weiteren Bestandteil, der ausgewählt ist aus: Magnesium, Kalium, Glukose, Aminosäuren, L-Arginin, Tetrahydrobiopterin (H4b), Vitamin B6, Vitamin B12, Vitamin C , w-3-Fettsäuren, entzündungshemmenden Wirkstoffen, Betablocker-Wirkstoffen, Adrenalin, Noradrenalin, alpha-adrenergischen, agonistischen Wirkstoffen, anti-vaskulären, endothelialen Wachstumsfaktor-(anti-VEGF)-Wirkstoffen, deren pharmazeutisch akzeptablen Salzen und den Mischungen davon;
zur gleichzeitigen oder getrennten Verwendung bei der Vorbeugung und / oder der Behandlung einer Krankheit, die ausgewählt ist aus Retinitis pigmentosa und der Stargardt-Krankheit.

10. Kit zur Verwendung gemäß Anspruch 9, wobei der erste wirksame Bestandteil und / oder der zweite wirksame Bestandteil und / oder der dritte wirksame Bestandteil, sofern vorhanden, auf demselben Weg oder auf unterschiedlichen Wegen verabreicht wird / werden.

11. Kit zur Verwendung gemäß irgendeinem der Ansprüche 9 bis 10, wobei wenigstens einer der wirksamen Bestandteile die Form einer ophthalmologischen Lösung oder Augentropfen oder Salbe aufweist.

12. Kit zur Verwendung gemäß irgendeinem der Ansprüche 9 bis 11, wobei
- Ramipril oder Ramiprilat einem Menschen topisch in einer Konzentration von 0,5 bis 5 % oder 0,5 bis 3 %, z. B., 0,5 %, 1 % oder 2 % und / oder oral in einer Menge von 0,5 bis 5 mg / Tag, bevorzugt 1 bis 2 mg / Tag, z. B. 1,25 mg / Tag verabreicht wird; und / oder
- Folsäure oder Folat einem Menschen topisch in einer Konzentration von 0,5 bis 5 % oder 0,5 bis 3 %, z. B. 0,5 %, 1 % oder 2 % und / oder oral in einer Menge von 2 bis 8 mg / Tag, bevorzugt 4 bis 6 mg / Tag, z. B. 5 mg / Tag verabreicht wird.

13. Kit zur Verwendung gemäß Anspruch 12, wobei Ramipril oder Ramiprilat und / oder Folsäure oder Folat topisch in Form von Augentropfen verabreicht wird / werden.

## Revendications

1. Composition destinée à être utilisée dans la prévention et/ou le traitement d'une maladie choisie parmi la rétinopathie pigmentaire et la maladie de Stargardt, **caractérisée en ce qu'**elle comprend :
a. un premier principe actif, qui consiste en un inhibiteur de l'enzyme de conversion de l'angiotensine (IECA) choisi dans le groupe consistant en : Ramipril, Ramiprilate et l'un de leurs sels pharmaceutiquement acceptables, et
b. un deuxième principe actif qui est choisi dans le groupe consistant en : acide folique, folate et un de leurs sels pharmaceutiquement acceptables, et
c. facultativement, au moins un autre principe actif choisi parmi les suivants : magnésium, potassium, glucose, acides aminés, L-arginine, tétrahydrobioptérine (H4b), vitamine B6, vitamine B12, vitamine C, acides gras w-3, agents anti-inflammatoires, agents bêta-bloquants, adrénaline, noradrénaline, agents agonistes alpha adrénergiques, agents anti-facteur de croissance de l'endothélium vasculaire (anti-VEGF), leurs sels pharmaceutiquement acceptables et leurs mélanges.

2. Composition destinée à être utilisée selon la revendication 1, dans laquelle le premier principe actif est le Ramipril et le second principe actif est l'acide folique, la composition comprenant, en outre, du magnésium ou un sel pharmaceutiquement acceptable de celui-ci et / ou du potassium ou un sel pharmaceutiquement acceptable de celui-ci.

3. Composition destinée à être utilisée selon la revendication 1 ou 2, dans laquelle la leucine est une acétyl-leucine et, en particulier, une N-acétyl-DL-leucine.

4. Composition destinée à être utilisée selon l'une quelconque des revendications 1 à 3, se présentant sous une forme appropriée pour une administration entérale, en particulier, orale, parentérale, en particulier, intraveineuse, intramusculaire ou sous-cutanée, transdermique ou topique, en particulier, pour une administration topique dans l'œil, y compris une injection intravitréenne, ou pour une administration subténonienne.

5. Composition destinée à être utilisée selon l'une quelconque des revendications 1 à 4, se présentant sous la forme d'un comprimé, d'une solution, d'une lotion, de gouttes, d'une crème ou d'une pommade.

6. Composition destinée à être utilisée selon l'une quelconque des revendications 1 à 5, se présentant sous la forme d'une solution ophtalmique ou de gouttes ophtalmiques.

7. Composition destinée à être utilisée selon l'une quelconque des revendications 1 à 6, dans laquelle :
- le Ramipril ou le Ramiprilate est administré par voie topique à une concentration de 0,5 à 5 % ou de 0,5 à 3 %, par exemple, 0,5 %, 1 % ou 2 %, et / ou par voie orale, dans une quantité de 0,5 à 5 mg / jour, de préférence, de 1 à 2 mg / jour, par exemple, 1,25 mg / jour chez l'homme ; et / ou
- l'acide folique ou le folate est administré par voie topique à une concentration de 0,5 à 5 % ou de 0,5 à 3 %, par exemple 0,5 %, 1 % ou 2 %, et/ou par voie orale, en une quantité de 2 à 8 mg/jour, de préférence 4 à 6 mg/jour, par exemple 5 mg/jour chez l'homme.

8. Composition destinée à être utilisée selon la revendication 7, dans laquelle le Ramipril ou le Ramiprilate et / ou l'acide folique ou le folate sont administrés par voie topique sous forme de gouttes oculaires.

9. Kit comprenant :
un premier principe actif, qui consiste en un inhibiteur de l'enzyme de conversion de l'angiotensine (IECA) choisi dans le groupe consistant en : Ramipril, Ramiprilate et l'un de leurs sels pharmaceutiquement acceptables, et
b. un deuxième principe actif qui est choisi dans le groupe consistant en : acide folique, folate, et
c. facultativement, au moins un autre principe actif choisi parmi les suivants : magnésium, potassium, glucose, acides aminés, L-arginine, tétrahydrobioptérine (H4b), vitamine B6, vitamine B12, vitamine C, acides gras w-3, agents anti-inflammatoires, agents bêta-bloquants, adrénaline, noradrénaline, agents agonistes alpha adrénergiques, agents anti-facteur de croissance de l'endothélium vasculaire (anti-VEGF), leurs sels pharmaceutiquement acceptables et leurs mélanges ;
pour une utilisation simultanée ou séparée dans la prévention et / ou le traitement d'une maladie choisie parmi la rétinopathie pigmentaire et la maladie de Stargardt.

10. Kit destiné à être utilisé selon la revendication 9, dans lequel le premier principe actif et / ou le deuxième principe actif et / ou le troisième principe actif, le cas échéant, sont administrés par la même voie ou par des voies différentes.

11. Kit destiné à être utilisé selon l'une quelconque des revendications 9 à 10, dans lequel au moins l'un des principes actifs se présente sous la forme d'une solution ophtalmique ou de gouttes ou de pommade pour les yeux.

12. Kit destiné à être utilisé selon l'une quelconque des revendications 9 à 11, dans lequel :
- le Ramipril ou le Ramiprilate est administré par voie topique à une concentration de 0,5 à 5 % ou de 0,5 à 3 %, par exemple, 0,5 %, 1 % ou 2 %, et / ou par voie orale, dans une quantité de 0,5 à 5 mg / jour, de préférence, de 1 à 2 mg / jour, par exemple, 1,25 mg / jour chez l'homme ; et / ou
- l'acide folique ou le folate est administré par voie topique à une concentration de 0,5 à 5 % ou de 0,5 à 3 %, par exemple 0,5 %, 1 % ou 2 %, et/ou par voie orale, en une quantité de 2 à 8 mg/jour, de préférence 4 à 6 mg/jour, par exemple 5 mg/jour chez l'homme.

13. Kit destiné à être utilisé selon la revendication 12, dans lequel le Ramipril ou le Ramiprilate et / ou l'acide folique ou le folate est(sont) administré(s) par voie topique sous forme de gouttes oculaires.
